# EUROPEAN PATENT SPECIFICATION

(11) **EP 2 348 022 B1**
(45) Date of publication and mention of the grant of the patent: **09.01.2013**
(21) Application number: 11002265.4
(22) Date of filing: 27.03.2006
(51) Int. Cl.: C07D 333/54, C07D 333/64, C07D 409/12

(54) **Process for production of 1-(3-(2-(1-benzothiophen-5-yl)ethoxy)propyl)azetidin-3-ol or salts thereof**
Prozess zur Herstellung von 1-(3-(2-(1-Benzothiophen-5-yl)-ethoxy)propyl)azetidin-3-ol sowie deren Salze
Procédé de fabrication de l'azétidin-3-ol 1-(3-(2-(1-benzothiophén-5-yl)-éthoxy)propyl) et de leurs sels

(30) Priority: 28.03.2005 JP 2005090831; 15.06.2005 JP 2005174738; 15.07.2005 JP 2005206808; 09.08.2005 JP 2005230666
(43) Date of publication of application: 27.07.2011
(62) Divisional of application: 10002020.5
(73) Proprietor: Toyama Chemical Co., Ltd., Shinjuku-ku Tokyo 160-0023 (JP)
(72) Inventor: Saitoh,Akihito, Toyama-shi Toyama (JP); Suzuki, Yoshiaki, Toyama-shi Toyama (JP); Yonezawa, Kenji, Toyama-shi Toyama (JP); Kawamura, Mitsuhide, Toyama-shi Toyama (JP); Kusanagi, Takahiko, Toyama-shi Toyama (JP); Nakai, Takashi, Toyama-shi Toyama (JP)
(74) Representative: Blodig, Wolfgang

(56) References cited:
- EP-A- 1 437 353
- EP-A- 1 614 419
- GORDON W GRIBBLE: "Sodium borohydride in carboxylic acid media: a phenomenal reduction system", CHEMICAL SOCIETY REVIEWS, CHEMICAL SOCIETY, LONDON, GB LNKD- DOI:10.1039/A827395Z, vol. 27, 1 January 1998 (1998-01-01), pages 395-404, XP007908370, ISSN: 0306-0012
- H. G. WEISS, I. SHAPIRO': "Diborane from the Sodium Borohydride-Sulfuric Acid Reaction", JOURNAL OF THE AMERICAN CHEMICAL SOCIETY, vol. 81, 1959, pages 6167-6168, XP002595830, DOI: 10.1021/ja01532a016

## Description

### FIELD OF THE INVENTION

The present invention relates to a new process for production of 1-(3-(2-(1-benzothiophen-5-yl)ethoxy)propyl)azetidin-3-ol or salts thereof which is useful as remedies for central nervous system and peripheral nerve diseases.

### BACKGROUND ART

1-(3-(2-(1-benzothiophen-5-yl)ethoxy)propyl)azetidin-3-ol or salts thereof has nerve protective action, nerve regeneration promotion action and neurite outgrowth action, and is a useful compound as remedies for central nervous system and peripheral nerve diseases.

As processes for production of this compound, for example, (1) a process of reacting 5-(2-(3-chloropropoxy)ethyl)-1-benzothiophene with 3-azetidinol or salts thereof, (2) a process of subjecting 1-(3-(2-(1-benzothiophen-5-yl)ethoxy) propionyl)azetidin-3-ol obtained from 3-(2-(1-benzothiophen-5-yl)ethoxy)propionic acid or its salts thereof to reduction reaction with borane-tetrahydrofuran complex or subjecting it to reduction reaction with sodium borohydride in the presence of boron trifluoride complex tetrahydrofuran complex, and so forth are known (patent document 1 defined below and EP 1 614 419 A1).

However, the process of (1) has the following defects, (A) the yield is low, (B) complicated procedures of purification such as silica gel column chromatography are necessary, (C) therefore, a lot of waste is egested, and so forth.

In addition, the process of (2) cannot be satisfied as an industrial manufacturing process, because the process has the following defects, (A) the process uses reagents of borane-tetrahydrofuran complex and boron trifluoride tetrahydrofuran and so forth, which are harmful to human body, highly flammable, highly toxic and having problems of stability, (B) therefore, attention is necessary to handle and store it and special equipments are required, and so forth.

Gordon Gribble, Chemical Society Reviews 27 (1998) 395-404 describes sodium borohydride in carboxylic acid media as a phenomenal reduction system. Weiss et al., J. Am. Chem. Soc. 81 (1959) 6167-6168 describes the production of diborane from the sodium borohydride-sulfuric acid reaction.

[patent document 1]
International publication No. 03/035,647 pamphlet, which corresponds to EP 1 431 353 A1.

### DISCLOSURE OF THE INVENTION

### PROBLEM TO BE SOLVED BY THE INVENTION

A new process for production of 1-(3-(2-(1-benzothiophen-5-yl)ethoxy)propyl)azetidin-3-ol and salts thereof, process which has safety to human body, low environmental loads and a possibility of mass production, is strongly expected.

### MEANS TO SOLVE THE PROBLEM

Under the circumstances, the present inventors have studied zealously and consequently, and found a process for production of 1-(3-(2-(1-benzothiophen-5-yl)ethoxy)propyl)azetidin-3-ol or salts thereof by subjecting 1-(3-(2-(1-benzothiophen-5-yl)ethoxy)propionyl)azetidin-3-ol to reduction reaction with an addition of activator in the presence of alkali metal borohydride wherein the activator is a protonic acid, and have completed the present invention.

### EFFECT OF THE INVENTION

The process for production of 1-(3-(2-(1-benzothiophen-5-yl)ethoxy)propyl)-3-azetidinol or salts thereof of the present invention has the following characteristics, (1) the yield is high, (2) silica gel column chromatography is not required, (3) therefore, the amount of wastes is small, (4) reagents which have harmfulness and problems of stability are not used, and so forth, and the process is useful as industrial manufacturing process.

### BEST MODE FOR CARRYING OUT THE INVENTION

The present invention is explained below in detail.

In the process for production of 1-(3-(2-(1-benzothiophen-5-yl)ethoxy)propyl)azetidin-3-ol or salts thereof by subjecting 1-(3-(2-(1-benzothiophen-5-yl)ethoxy)propionyl)azetidin-3-ol to reduction reaction with an addition of activator in the presence of alkali metal borohydride, the process for production in which the alkali metal borohydride used is sodium borohydride is preferable.
The process for production in which the protonic acid activator is sulfuric acid or hydrogen chloride and so forth is preferable, and the process for production in which the activator used is sulfuric acid is more preferable.
In the case that the activator is sulfuric acid, the process for production in which the volume of sulfuric acid used is 0.5-0.6 times mole per mole of the alkali metal borohydride, the addition of sulfuric acid at 0 to 30°C for 10 minutes to 6 hours and the subsequent reaction at 30 to 70°C is preferable.

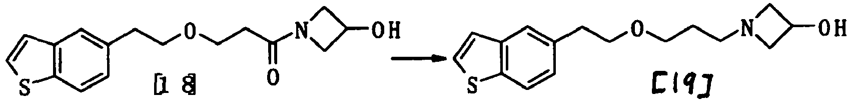

1-(3-(2-(1-benzothiophen-5-yl)ethoxy)propyl)azetidin-3-ol of the compound of the formula [19] or salts thereof can be produced by subjecting the compound of the formula [18] to reduction reaction with an addition of a protonic acid activator in the presence of alkali metal borohydride.

This reaction is usually carried out in the presence of solvent, the solvent used, if it does not affect an influence on the reaction, is not particularly limited, but for example, ethers such as tetrahydrofuran, 1,2-dimethoxyethane, bis(2-methoxyethyl)ether, dioxane and the like; is given, and tetrahydrofuran is more preferable.

In addition, these solvents may be used in admixture of halogenated hydrocarbons such as dichloromethane, chloroform and the like;
aromatic hydrocarbons such as benzene, toluene, xylene and the like;
and aliphatic hydrocarbons such as hexane, cyclohexane, octane and the like.
The amount of the solvent used is preferably 1 to 20 times volume per weight of the compound of the formula [18], and is more preferably 3 to 10 times(v/w).

As the alkali metal borohydride used in this reaction, for example, sodium borohydride, lithium borohydride, potassium borohydride and the like; are given, and sodium borohydride is preferable.
The amount of the alkali metal borohydride is preferably 1 to 10 times mole per mole of the compound of the formula [18], and is more preferably 2 to 3 times mole.

As the protonic acid activator used in this reaction, for example, sulfuric acid, hydrogen chloride, trifluoroacetic acid and the like are given.
As a preferable activator, sulfuric acid, hydrogen chloride and the like; is given, and sulfuric acid is more preferable.
The amount of the activator used is different in the kind of the activator, but for example, in the case of sulfuric acid, it is preferably 0.5 to 1 times mole per mole of alkali metal borohydride, and is more preferably 0.5 to 0.6 times mole.
In addition, the addition time of the activator is different in the kind of the activator, but in the case of sulfuric acid, it is preferably for 10 minutes to 6 hours, and is more preferably for 30 minutes to 4 hours.
In addition, the activator may be dissolved in solvent appropriately, and the dissolved solvent may be added.

Further, in the case that the amount of the alkali metal borohydride is 2.0 to 2.2 times mole per mole of the compound of the formula [18] and the amount of sulfuric acid is 0.5 to 0.6 times mole per mole of the alkali metal borohydride and the dropping time of the sulfuric acid is 1 to 4 hours, the compound of the formula [19] or salts thereof having high purity can be obtained because of the further suppression of the formation of by-products.

The reaction temperature is not particularly limited, but may be -20 to 150°C, and is preferably 0 to 70°C.
After an addition of activator at 0 to 30°C, it is preferable to react at 30 to 70°C, and after an addition of activator at 0 to 30°C, it is more preferable to react at 40 to 60°C.
The reaction time is not particularly limited, but may be for 10 minutes to 50 hours, is preferably for 1 to 20 hours.

In the present invention, as a preferable process for production, the following process is given,
the process by suspending the compound of the formula [18] in ether (3-10 times(v/w)), adding alkali metal borohydride(2 to 3 times mole), adding activator at 0 to 30°C, and subsequently reacting at 30 to 70°C for 1 to 20 hours is preferable, the process by suspending the compound of the formula [18] in ether (3 to 10 times(v/w)), adding sodium borohydride (2 to 3 times mole), adding protonic acid (0.5 to 1 times mole per mole of sodium borohydride) at 0 to 30°C, and subsequently reacting at 30 to 70°C for 1 to 20 hours is more preferable, and the process by suspending the compound of formula [18] in tetrahydrofuran (3 to 10 times(v/w)), adding sodium borohydride (2.0 to 2.2 times mole), adding sulfuric acid (0.5 to 0.6 times mole per mole of sodium borohydride) at 0 to 30°C for 1 to 4 hours, and subsequently reacting at 40 to 60°C for 1 to 20 hours is further preferable.

After the termination of the reaction, the compound of the formula [19] or salts thereof obtained in this way can be isolate in usual manner.
For example, after the termination of the reaction, it can be isolated by adding 6.0mol/L hydrochloric acid to decompose an excessive reducing agent, cooling to room temperature, subsequently making the reaction mixture alkaline with aqueous sodium hydroxide, extracting with organic solvent such as ethyl acetate, and subsequently removing the solvent from the extract.
In addition, it can be isolated as a salt by an addition of acid into the extract.

As the salt of the compound of the formula [19], the salt, if it is usually known for a salt in basic group such as amino group, is not particularly limited, but for example, salts with mineral acid such as hydrochloric acid, hydrobromic acid, nitric acid, sulfuric acid and the like;
salts with organic carboxylic acid such as formic acid, acetic acid, citric acid, oxalic acid, fumaric acid, maleic acid, succinic acid, malic acid, tartaric acid, aspartic acid, trichloroacetic acid, trifluoroacetic acid and the like;
and salts with sulfonic acid such as methanesulfonic acid, benzenesulfonic acid, toluenesulfonic acid, mesitylene sulfonic acid, naphthalenesulfonic acid and the like; are given.
As a preferable salt, a pharmacologically acceptable salt is given, and a salt with maleic acid is more preferable.

In the compound produced in the present invention, if isomer (for example, optical isomer), hydrate, solvate and various kinds of crystalline forms exist, these all may be produced.
In addition, in the compound used by the process for production described above, if isomer (for example, optical isomer), hydrate, solvate and various kinds of crystal form exist, all these can be used in the process for production of the present invention.

### Examples

Next, the present invention will be described in the following reference examples and examples. Examples 1 to 5, 6-1, 6-2, and 6-4 are reference examples.
The mixing ratios in the eluents are by volume. A case without description in particular, the carrier in silica gel column chromatography is B.W. silica gel, BW-127ZH or PSQ100B(product of Fuji Silysia Chemical Ltd.).
The abbreviations in examples mean the following: Me: methyl, Et: ethyl, Pr: propyl, Bu: butyl, ^{t}Bu: tert-butyl DMSO-d₆: dimethylsulfoxide-d₆

### Reference Example 1

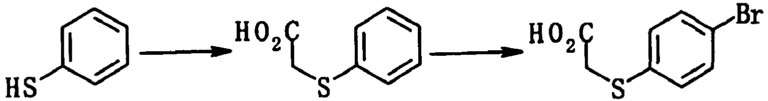

To water(275mL) suspension of 546g of thiophenol was dropwise added water(550mL) solution of 585g of potassium hydroxide at no more than 20°C. Next, thereto was dropwise added a water(825mL) solution of 492g of chloroacetic acid, which was then stirred at 80 to 90°C for 3 hours. After cooling the reaction mixture, the pH was adjusted to 1.5 with hydrochloric acid, and thereto were added 1650mL of dichloromethane and 550mL of water. The organic layer was separated, and anhydrous magnesium sulfate was added. Insoluble matter was filtered off, and to the filtrate was added 5.95g of iron(III) chloride, and dropwise added 832g of Bromine at 5 to 10°C, which was then stirred at room temperature for 5 hours. After cooling the reaction solution to 5°C, thereto was dropwise added water(825mL) solution of 187g of sodium sulfite, and the pH was adjusted 1.2 with hydrochloric acid. After stirring at 5 to 10°C for 1 hour, the precipitate was collected by filtration to provide solid matter. To this solid matter was added 2000mL of toluene, from which water was removed by heating and azeotropic distillation. The reaction mixture was cooled to 5°C over a period of 2 hours. After stirring at the same temperature for 1 hour, crystals precipitate was collected by filtration to provide 1108g of (4-bromophenylthio)acetic acid as white solid form.
¹H-NMR(CDCl₃) δ value: 3.65(2H,s),7.25-7.35(2H,m),7.40-7.50(1H,m)

### Reference Example 2

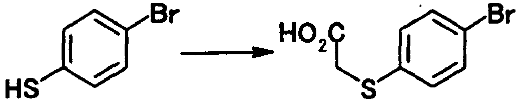

To water(600mL) solution of 88.9g of sodium hydroxide was added 200g of 4-bromothiophenol and dropwise added water(300mL) solution of 105g of chloroacetic acid, which was then stirred at 60 to 70°C for 1 hour. After cooling the reaction mixture to 40°C, thereto were added 140mL of hydrochloric acid and 600mL of toluene, which was then heated to 80°C. The organic layer was separated and slowly cooled to 5°C. After stirring at the same temperature for 1 hour, the crystals precipitate was collected by filtration to provide 243g of (4-bromophenylthio)acetic acid as white solid form.
The Chemical shift values of ¹H-NMR spectral in CDCl₃ agreed with the values of reference example 1.

### Example 1-1

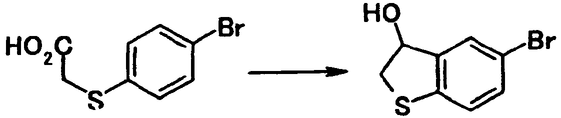

To dichloromethane(750mL) suspension of 250g of (4-bromophenylthio)acetic acid were added 2.5mL of N, N-dimethylformamide and 132g of thionyl chloride, which was then refluxed for 1 hour. After cooling the reaction mixture to 20°C, thereto was dropwise added dichloromethane(1500mL) suspension of 148g of aluminum chloride at 5 to 15°C, which was then stirred at 15 to 25°C for 1.5 hours. Next, this reaction mixture was added dropwise to a mixed solution of 1310mL of water and 188mL of hydrochloric acid under cooling. The organic layer was separated, thereto was added 1250mL of water, and the pH was adjusted to 3.0 with 5% potassium carbonate aqueous solution. The organic layer was separated and cooled to 5°C. Thereto were added 15.3g of sodium borohydride and 500mL of methanol, which was then stirred at 10 to 20°C for 2 hours. To the reaction solution was added 750mL of water, followed by adjustment to pH7.0 using acetic acid and left unattended in room temperature overnight. To the reaction solution was added 200mL of 5% potassium hydroxide aqueous solution, and the organic layer was separated. To the organic layer was 25.0g of activated carbon, which was then stirred at room temperature. Insoluble matter was filtered off, and the solvent of filtrate was distilled off. To the resultant residue was added cyclohexane, the crystals precipitated were collected by filtration to provide 194g of 5-bromo-2,3-dihydro-1-benzothiophen-3-ol as pale red solid form.
¹H-NMR (CDCl₃) δ value:
2.18(1H,d,J=8.3Hz),3.30(1H,dd,J=12.0,4.4Hz),3.61(1H,dd, J=12.0,6.3Hz),5.30-5.40(1H,m),7.11(1H,d,J=8.3Hz), 7.35(1H,dd,J=8.3,2.0Hz),7.50(1H,d,J=2.0Hz)

### Example 1-2

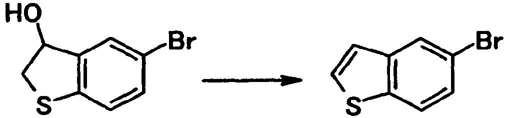

To acetone(600mL) solution of 300g of 5-bromo-2,3-dihydro-1-benzothiophen-3-ol was added 12.4g of p-toluenesulfonic acid monohydrate, which was then refluxed for 2 hours. To the reaction solution was added 15.0g of activated carbon, which was then stirred. Insoluble matter was filtered off and washed with 300mL of acetone. The filtrate and washings were combined, to which was dropwise added 2700mL of water at 5 to 15°C. The precipitate was collected by filtration to provide 268g of 5-bromo-1-benzothiophene as pale purple solid form.
¹H-NMR(CDCl₃) δ value:
7.27(1H,d,J=5.4Hz),7.44(1H,dd,J=8.5,1.9Hz),7.48(1H,d,J= 5.4Hz),7.74(1H,d,J=8.5Hz),7.97(1H,d,J=1.9Hz)

### Example 2-1

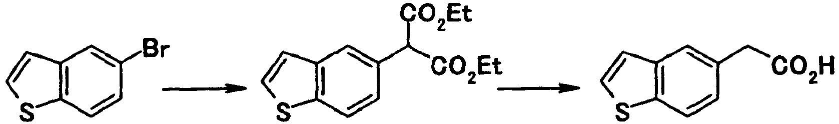

(1) To 1,2-dimethoxyethane(10mL) suspension of 0.02g of tris(dibenzylideneacetone)dipalladium(0) were added 0.11g of 10%(w/w)tri(tert-butyl)phosphine/hexane, 1.76g of cesium carbonate, 0.50g of 5-bromobenzothiophene and 0.45g of diethyl malonate, which was then refluxed for 2 hours. To the reaction mixture were added water and ethyl acetate, followed by adjustment to pH2 using 2mol/L hydrochloric acid. The organic layer was separated, and dried over anhydrous magnesium sulfate, followed by distilling off the solvent under reduced pressure. The resultant residue was purified using silica gel column chromatography (eluent; hexane:ethyl acetate = 10:1) to provide 0.69 g of diethyl 2-(1-benzothiophen-5-yl)malonate as white solid form.
   ¹H-NMR(CDCl₃) δ value:
   1.27(6H,t,J=7.1Hz),4.1-4.3(4H,m),4.73(1H,s),7.33 (1H,d,J=5.4Hz),7.40(1H,dd,J=8.3,2.0Hz),7.45(1H,d,J=5.4H z),7.87(1H,d,J=8.3Hz),7.87(1H,d,J=2.0Hz) (2) To ethylene glycol(1.0mL) suspension of 0.25g of diethyl 2-(1-benzothiophen-5-yl)malonate were added 1.0mL of 40%(w/w) potassium hydroxide aqueous solution and 0.3mL of water, which was then refluxed for 2 hours. To the reaction mixture were added water and toluene, and the aqueous layer was separated. The pH was adjusted to 2 with 6mol/L hydrochloric acid, and thereto was added ethyl acetate. The organic layer was separated, and dried over anhydrous magnesium sulfate, followed by distilling off the solvent under reduced pressure. The resultant residue was suspended to 5mL of xylene, and thereto was added 0.01g of p-toluenesulfonic acid monohydrate, which was then refluxed for 30 minutes. The solvent was distilled off under reduced pressure, and to the resultant residue were added toluene and cyclohexane. The precipitate was collected by filtration to provide 0.02g of 2-(1-benzothiophen-5-yl)acetic acid as pale yellow solid form.
   ¹H-NMR(CDCl₃) δ value:
   3.76(2H,s),7.2-7.3(1H,m),7.29(1H,d,J=5.4Hz), 7.44(1H,d,J=5.4Hz),7.73(1H,s),7.83(1H,d,J=8.1Hz)

### Example 2-2

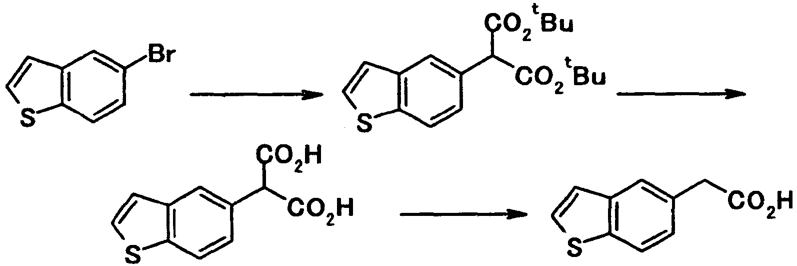

(1) To 1,2-dimethoxyethane(10mL) solution of 0.11g of 10%(w/w)tri(tert-butyl)phosphine/hexane were added 0.02g of tris(dibenzylideneacetone)dipalladium(0), 1.76g of cesium carbonate, 0.50g of 5-bromobenzothiophene and 0.61g of tert-butyl malonate, which was then refluxed for 2 hours. Next, thereto were added 0.02g of tris(dibenzylideneacetone)dipalladium(0) and 0.11g of 10%(w/w)tri(tert-butyl)phosphine/hexane, which was then refluxed for 1 hour. This reaction mixture was added to a mixed solution of 30mL of water and 20mL of ethyl acetate, and the pH was adjusted to 3 with 6mol/L hydrochloric acid. The organic layer was separated, and dried over anhydrous magnesium sulfate, followed by distilling off the solvent under reduced pressure. The resultant residue was purified using silica gel column chromatography (eluent; hexane: ethyl acetate = 20:1) to provide 0.49 g of di(tert-butyl) 2-(1-benzothiophen-5-yl)malonate as white solid form.
   ¹H-NMR(CDCl₃) δ value:
   1.47(18H,s),4.55(1H,s),7.32(1H,d,J=5.4Hz),7.39(1H,dd,J= 8.5,1.7Hz),7.43(3H,d,J=5.4Hz),7.84(1H,d,J=1.7Hz),7.86(1 H,d,J=8.5Hz)
(2) To toluene(2.5mL) solution of 0.25g of di(tert-butyl) 2-(1-benzothiophen-5-yl)malonate was added 0.01g of p-toluenesulfonic acid monohydrate, which was then refluxed for 1 hour. After cooling the reaction mixture, the precipitate was collected by filtration to provide 0.14g of 2-(1-benzothiophen-5-yl)malonic acid as white solid form.
   ¹H-NMR(CDCl₃) δ value:
   4.80(1H,s),7.3-7.5(1H,m),7.47(1H,d,J=5.5Hz), 7.77(1H,d,J=5.5Hz),7.89(1H,s),7.97(1H,d,J=8.3Hz)
(3) To xylene(2mL) suspension of 0.10g of 2-(1-benzothiophen-5-yl)malonic acid was added 4mg of p-toluenesulfonic acid monohydrate, which was then refluxed for 1 hour. The solvent was distilled off under reduced pressure, and to the resultant residue was added cyclohexane. The precipitate was collected by filtration to provide 0.08g of 2-(1-benzothiophen-5-yl)acetic acid as white solid form.

The Chemical shift values of ¹H-NMR spectral in CDCl₃ agreed with the values of example 2-1(2).

### Example 2-3

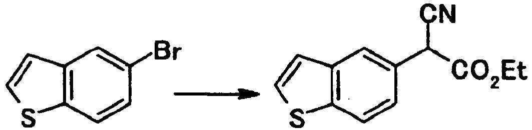

To toluene(3mL) solution of 0.21g of ethyl cyanoacetate were added 0.41g of potassium tert-butoxide, 0.30g of 5-bromobenzothiophene and 0.02g of tetrakis(triphenylphosphine)palladium(0), which was then refluxed for 7.5 hours. To the reaction mixture was added water, and the pH was adjusted to 2 with hydrochloric acid. Thereto was added ethyl acetate, and insoluble matter was filtered off. The organic layer was separated, washed with water, and dried over anhydrous magnesium sulfate, followed by distilling off the solvent under reduced pressure. The resultant residue was purified using silica gel column chromatography (eluent; hexane:ethyl acetate = 5:1) to provide 0.16g of ethyl 2-(1-benzothiophen-5-yl)-2-cyanoacetate as pale yellow solid form.
¹H-NMR(CDCl₃) δ value:
1.29(3H,t,J=7.1Hz),4.25(2H,m),4.84(1H,s),7.37(1H,d,J=5. 4Hz),7.41(1H,dd,J=8.5,1.7Hz),7.54(1H,d,J=5.4Hz),7.92(1H ,d,J=8.5Hz),7.94(1H,d,J=1.7Hz)

### Example 2-4

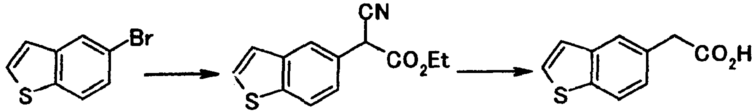

To toluene(25mL) suspension of 0.16g of dichlorobis(triphenylphosphine)palladium(II) were added 0.12g of triphenylphosphine, 0.01g of sodium borohydride, 5.79g of potassium tert-butoxide and 2.92g of ethyl cyanoacetate, which was then stirred at room temperature for 10 minutes. Thereto were added 5.00g of 5-bromobenzothiophene and 25mL of toluene, which was then refluxed for 4 hours. Thereto was added 0.14g of tetrakis(triphenylphosphine)palladium(0), which was then refluxed for 2 hours. Next, to the reaction solution were added 25mL of ethanol, 2.82g of sodium hydroxide and 5mL of water, which was then refluxed for 6 hours. Thereto was added 2.82g of sodium hydroxide, which was then refluxed for 5 hours. To the reaction mixture were added 15mL of water and 0.5g of activated carbon, and insoluble matter was filtered off. The aqueous layer was separated, and to the solution was added 35mL of ethanol. The pH was adjusted to 2 with 15mL of hydrochloric acid. Thereto was added 15mL of water, which was then stirred at 40°C. Thereto was added 30mL of water, which was stirred. After that, it was cooled. The precipitate was collected by filtration to provide 3.38g of 2-(1-benzothiophen-5-yl)acetic acid as pale yellow solid form.

The Chemical shift values of ¹H-NMR spectral in CDCl₃ agreed with the values of example 2-1(2).

### Example 2-5

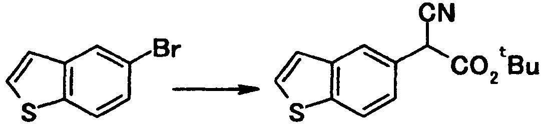

To 1,2-dimethoxyethane(25mL) suspension of 0.16g of dichlorobis(triphenylphosphine)palladium(II) were added 0.12g of triphenylphosphine, 0.01g of sodium borohydride, 5.53g of potassium tert-butoxide and 3.48g of tert-butyl cyanoacetate, which was then stirred at room temperature for 10 minutes. Thereto was added 5.00g of 5-bromobenzothiophene, which was then refluxed for 2 hours. After to the reaction mixture was added 15mL of water, the pH was adjusted to 1 with 2mL of hydrochloric acid. The precipitate was collected by filtration to provide 5.69g of tert-butyl 2-(1-benzothiophen-5-yl)-2-cyanoacetate as pale yellow solid form.
¹H-NMR(CDCl₃) δ value:
1.45(9H,s),4.73(1H,s),7.36(1H,d,J=5.6Hz),7.39(1H,dd,J=8 .5,2.0Hz),7.52(1H,d,J=5.6Hz),7.91(1H,d,J=8.5Hz),7.9-8.0(1H,m)

### Example 2-6

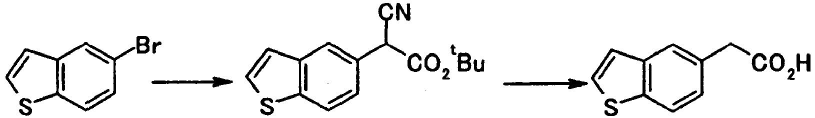

To 1,2-dimethoxyethane(1.00L) solution of 250g of 5-bromobenzothiophene were added 276g of potassium tert-butoxide and 174g of tert-butyl cyanoacetate. Thereto were added 8.23g of dichlorobis(triphenylphosphine)palladium(II) and 6.15g of triphenylphosphine at 80 to 85°C, which was then refluxed for 2 hours. Next, to the reaction mixture were added 500mL of ethylene glycol, 250mL of water and 263g of potassium hydroxide, which was then refluxed for 4 hours. To the reaction mixture were added 1.50L of water and 12.5g of kieselguhr (cellpure, product of Advanced Minerals Company). After insoluble matter was filtered off, to the filtrate was added 250mL of toluene, and the aqueous layer was separated. To the aqueous layer were added 375mL of toluene and 375mL of ethyl acetate, the pH was adjusted to 1 with 505mL of hydrochloric acid, and the organic layer was separated. The organic layer was treated with 12.5g of activated carbon. The solvent was distilled off under reduced pressure, to which was added toluene. The precipitate was collected by filtration to provide 176g of 2-(1-benzothiophen-5-yl)acetic acid as white solid form.

The Chemical shift values of ¹H-NMR spectral in CDCl₃ agreed with the values of example 2-1(2).

### Example 2-7

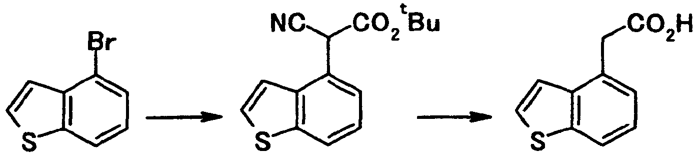

To 1,2-dimethoxyethane(3mL) solution of 0.30g of 4-bromobenzothiophene were added 0.33g of potassium tert-butoxide, 0.21g of tert-butyl cyanoacetate, 0.01g of dichlorobis(triphenylphosphine)palladium(II) and 0.01g of triphenylphosphine, which was then refluxed for 40 minutes. Thereto were added 0.33g of potassium tert-butoxide, 0.01g of dichlorobis(triphenylphosphine) palladium(II) and 0.01g of triphenylphosphine, which was then refluxed for 30 minutes. The reaction mixture was added to a mixed solution of water and ethyl acetate, and the pH was adjusted to 1 with 6mol/L hydrochloric acid. The organic layer was separated, and dried over anhydrous magnesium sulfate, followed by distilling off the solvent under reduced pressure. The resultant residue was purified using silica gel column chromatography (eluent; hexane:ethyl acetate = 5:1) to provide 0.26g of tert-butyl 2-(1-benzothiophen-4-yl)-2-cyanoacetate as pale brown oily form.
¹H-NMR(CDCl₃) δ value:
1.42(9H,s),5.03(1H,s),7.39(1H,t,J=7.8Hz),7.49(1H,d,J=7. 8Hz),7.54(1H,d,J=5.6Hz),7.59(1H,d,J=5.6Hz),7.92(1H,d,J= 7.8Hz)

(2) To ethylene glycol(1.0mL) solution of 0.25g of tert-butyl 2-(1-benzothiophen-4-yl)-2-cyanoacetate were added 1.0mL of aqueous solution of 40%(w/w)potassium hydroxide and 0.3mL of water, which was then stirred at 95 to 105°C for 1 hour. To the reaction mixture were added water and toluene, and the aqueous layer was separated. The pH was adjusted to 2 with 6mol/L hydrochloric acid, to which was added ethyl acetate. The organic layer was separated, and dried over anhydrous magnesium sulfate, followed by distilling off the solvent under reduced pressure. To the resultant residue was added cyclohexane. The precipitate was collected by filtration to provide 0.15g of 2-(1-benzothiophen-4-yl)acetic acid as white solid form.
   ¹H-NMR(CDCl₃) δ value:
   3.95(2H,s),7.2-7.4(2H,m),7.41(1H,d,J=5.5Hz), 7.47(1H,d,J=5.5Hz),7.82(1H,d,J=7.8Hz)

### Example 3-1

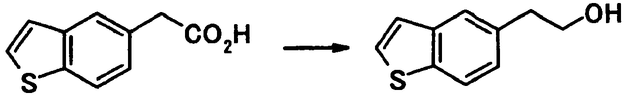

To 340mL of tetrahydrofuran was suspended 50.2g of sodium borohydride, to which were dropped tetrahydrofuran(340mL) solution of 170g of (1-benzothiophen-5-yl)acetic acid, and 65.1g of sulfuric acid successively, which was then stirred at room temperature for 2.5 hours. To this reaction mixture was dropwise added 85mL of acetone, which was then stirred for 30 minutes. Thereto were added 510mL of water and 680mL of toluene. The organic layer was separated, to which was added 510mL of water, and the pH was adjusted to 12 with 48mL of 20%(w/w)sodium hydroxide aqueous solution. The organic layer was separated, and washed with water, followed by distilling off the solvent. Thereto were added cyclohexane and toluene. The precipitate was collected by filtration to provide 135g of 2-(1-benzothiophen-5-yl)ethanol as white solid form.
¹H-NMR (CDCl₃) δ value:
1.41(1H,t,J=6.0Hz),2.99(2H,t,J-6.5Hz),3.8-4.0(2H,m), 7.22(1H,dd,J=8.3,1.7Hz),7.30(1H,d,J=5.4Hz),7.44(1H,d,J= 5.4Hz),7.6-7.7(1H,m),7.83(1H,d,J=8.3Hz)

### Example 3-2

To 5mL of 1,2-dimethoxyethane was suspended 2.95g of sodium borohydride, to which were dropwise added 1,2-dimethoxyethane(25mL) solution of 10g of (1-benzothiophen-5-yl)acetic acid, and 11mL of 6.9mol/L hydrochloride/1,2-dimethoxyethane solution, which was then stirred at room temperature for 1 hour. To this reaction mixture was dropwise added 5mL of acetone, which was then stirred for 30 minutes. Thereto were added 20mL of water, 30mL of toluene and 2mL of 2mol/L hydrochloric acid. Next, after the pH was adjusted to 9.5 with 20mL of 2mol/L sodium hydroxide aqueous solution, the organic layer was separated. The organic layer was dried over anhydrous magnesium sulfate, followed by distilling off the solvent. Thereto were added cyclohexane and toluene. The precipitate was collected by filtration to provide 7.84g of 2-(1-benzothiophen-5-yl)ethanol as white solid form.

### Example 3-3

To 40mL of tetrahydrofuran was suspended 4.72g of sodium borohydride, to this solution were dropwise added tetrahydrofuran(60mL) solution of 20g of (1-benzothiophen-5-yl)acetic acid, and 6.12g of sulfuric acid. The solution was heated to 66°C, followed by distilling off about 40mL of the solvent under normal pressure, which was then stirred at same temperature for 1 hour. After cooling, to this reaction mixture was dropwise added 10mL of acetone, which was then stirred for 30 minutes. Thereto were added 90mL of water and 80mL of toluene. The organic layer was separated, to which was added 60mL of water, and the pH was adjusted to 13.6 with 5mL of 5mol/L sodium hydroxide aqueous solution. The organic layer was separated, washed with water, followed by distilling off the solvent, and thereto were added cyclohexane and toluene. The precipitate was collected by filtration to provide 16.5g of 2-(1-benzothiophen-5-yl)ethanol as white solid form.

The Chemical shift values of ¹H-NMR spectral in CDCl₃ agreed with the values of example 3-1.

### Example 4-1

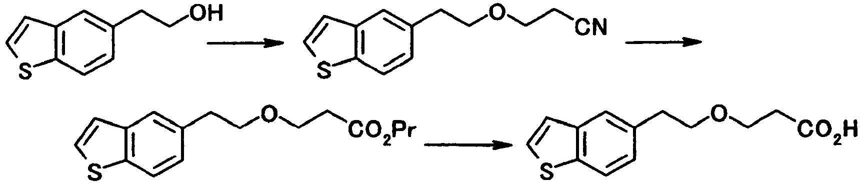

(1) To toluene(5mL) suspension of 0.23g of 40%(w/w)benzyltrimethylammonium hydroxide aqueous solution was added 5.00g of 2-(1-benzothiophen-5-yl)ethanol, and dropwise added 2.20mL of acrylonitrile at 0 to 5°C, which was then stirred at 0 to 20°C for 1 hour. To this reaction mixture was added 0.125mL of hydrochloric acid. Thereto were added 10mL of propanol, 1.0mL of water and 3.1mL of sulfuric acid, which was then refluxed for 6.5 hours. After cooling, to the reaction mixture were added 10mL of water and 10mL of toluene. The organic layer was separated, and dried over anhydrous magnesium sulfate. After insoluble matter was filtered off, the solvent was distilled off under reduced pressure. The resultant residue was purified using silica gel column chromatography (eluent; hexane:ethyl acetate = 15:1 to 7:1) to provide 7.21g of propyl 3-(2-(1-benzothiophen-5-yl)ethoxy)propionate as colorless oily form.
   ¹H-NMR(CDCl₃) δ value:
   0.91(3H,t,J=7.4Hz),1.57-1.67(2H,m),2.58(2H,t,J=6.4Hz), 2.99(2H,t,J=7.1Hz),3.71(2H,t,J=7.1Hz),3.74(2H,t,J=6.4Hz ),4.02(2H,t,J=6.7Hz),7.20(1H,dd,J=8.2,1.6Hz),7.28(1H,d, J=5.6Hz),7.41(1H,d,J=5.6Hz),7.60-7.70(1H,m),7.78 (1H,d,J=8.2Hz)
(2) To methanol(12mL) solution of 12.0g of propyl 3-(2-(1-benzothiophen-5-yl)ethoxy)propionate was added water(12mL) solution of 2.76g of potassium hydroxide, which was then stirred at room temperature for 1.5 hours. This reaction mixture was distilled off under reduced pressure, to which were added 36mL of toluene and 36mL of water. The pH was adjusted to 1.9 with 8mL of 6mol/L hydrochloric acid. The organic layer was separated, followed by distilling off the solvent under reduced pressure. Thereto were added 12mL of toluene and 24mL of cyclohexane. The precipitate was collected by filtration to provide 8.91g of 3-(2-(1-benzothiophen-5-yl)ethoxy)propionic acid as white solid form.
   ¹H-NMR(CDCl₃) δ value:
   2.63(2H,t,J=6.2Hz),3.00(2H,t,J=7.1Hz),3.72(2H,t,J=7.1Hz ),3.74(2H,t,J=6.2Hz),7.20(1H,dd,J=8.4,1.6Hz),7.27(1H,dd ,J=5.5,0.6Hz),7.40(1H,d,J=5.5Hz),7.65-7.70(1H,m),7.78 (1H,d,J=8.4Hz)

### Example 4-2

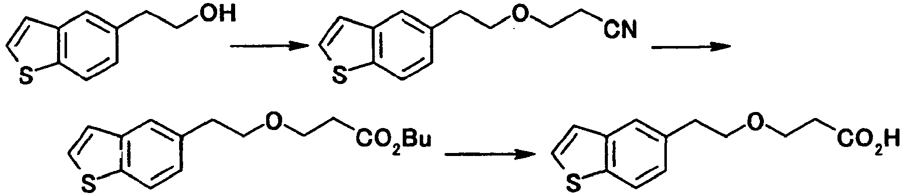

(1) To toluene(5mL) suspension of 5.00g of 2-(1-benzothiophen-5-yl)ethanol were added 0.23g of 40%(w/w)benzyltrimethylammonium hydroxide aqueous solution and 2.28mL of tetrahydrofuran, and dropwise added 2.20mL of acrylonitrile at 0 to 10°C, which was then stirred at same temperature for 1.5 hours. To this reaction mixture were added 0.1mL of hydrochloric acid, 10mL of butanol and 5mL of 50%(w/w) sulfuric acid, which was then refluxed for 15 hours. After cooling, to the reaction mixture was added 15mL of water. The organic layer was separated, and dried over anhydrous magnesium sulfate. After insoluble matter was filtered off, the solvent was distilled off under reduced pressure. The resultant residue was purified using silica gel column chromatography(eluent; hexane:ethyl acetate = 10:1) to provide 6.65g of butyl 3-(2-(1-benzothiophen-5-yl)ethoxy)propionate as colorless oily form.
   ¹H-NMR(CDCl₃) δ value:
   0.92(3H,t,J=7.4Hz),1.30-1.45(2H,m),1.50-1.65(2H,m),2.57 (2H,t,J=6.3Hz),2.99(2H,t,J=7.1Hz),3.71(2H,t,J=7.1Hz),3. 74(2H,t,J=6.3Hz),4.06(2H,t,J=6.7Hz),7.21(1H,dd,J=8.3,1. 7Hz),7.28(1H,dd,J=5.4,0.7Hz),7.41(1H,d,J=5.4Hz),7.65-7.70(1H,m),7.78(1H,d,J=8.3Hz)
(2) To methanol(5mL) solution of 5.00g of butyl 3-(2-(1-benzothiophen-5-yl)ethoxy)propionate was added water(5mL) solution of 1.10g of potassium hydroxide, which was then stirred at room temperature for 2 hours. This reaction mixture was distilled off under reduced pressure, to which were added 30mL of toluene and 30mL of water. The pH was adjusted to 1.6 with 3.5mL of 6mol/L hydrochloric acid. The organic layer was separated, followed by distilling off the solvent under reduced pressure. Thereto were added 15mL of toluene and 30mL of cyclohexane. The precipitate was collected by filtration to provide 3.60g of 3-(2-(1-benzothiophen-5-yl)ethoxy)propionic acid as white solid form.

The Chemical shift values of ¹H-NMR spectral in CDCl₃ agreed with the values of example 4-1(2).

### Example 4-3

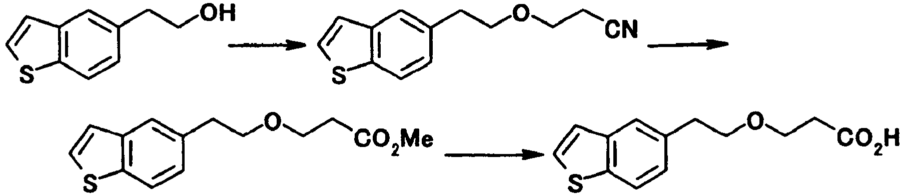

(1) To toluene(5mL) suspension of 5.00g of 2-(1-benzothiophen-5-yl)ethanol were added 0.23g of 40%(w/w)benzyltrimethylammonium hydroxide aqueous solution and 2.28mL of tetrahydrofuran, and dropwise added 2.22mL of acrylonitrile at 5°C, which was then stirred at 0 to 15°C for 1.5 hours. To this reaction mixture were added 0.13mL of hydrochloric acid, 10mL of methanol and 1.52g of water. Thereto was introduced 9.47g of hydrogen chloride, which was then refluxed for 4 hours. After cooling, to the reaction mixture were added 15mL of water and 10mL of toluene. The organic layer was separated, and dried over anhydrous magnesium sulfate. After insoluble matter was filtered off, the solvent was distilled off under reduced pressure. The resultant residue was purified using silica gel column chromatography(eluent; hexane:ethyl acetate = 5:1) to provide 7.36g of methyl 3-(2-(1-benzothiophen-5-yl)ethoxy)propionate as colorless oily form.
   ¹H-NMR(CDCl₃) δ value:
   2.58(2H,t,J=6.4Hz),2.99(2H,t,J=7.1Hz),3.65(3H,s),3.71(2 H,t,J=7.1Hz),3.74(2H,t,J=6.4Hz),7.20(1H,dd,J=8.3,1.7Hz) ,7.28(1H,d,J=5.4Hz),7.41(1H,d,J=5.4Hz),7.65-7.70(1H,m), 7.78(1H,d,J=8.3Hz)
(2) To methanol(5mL) solution of 5.00g of methyl 3-(2-(1-benzothiophen-5-yl)ethoxy)propionate was added water(5mL) solution of 1.27g of potassium hydroxide, which was then stirred at room temperature for 2 hours. This reaction mixture was distilled off under reduced pressure, to which were added 30mL of toluene and 30mL of water. The pH was adjusted to 1.0 with 5mL of 6mol/L hydrochloric acid. The organic layer was separated, followed by distilling off the solvent under reduced pressure. Thereto were added 11mL of toluene and 30mL of cyclohexane. The precipitate was collected by filtration to provide 4.51g of 3-(2-(1-benzothiophen-5-yl)ethoxy)propionic acid as white solid form.

The Chemical shift values of ¹H-NMR spectral in CDCl₃ agreed with the values of example 4-1(2).

### Example 4-4

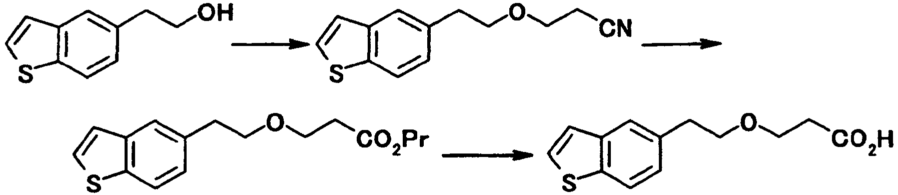

To toluene(50mL) suspension of 50.0g of 2-(1-benzothiophen-5-yl)ethanol was added 2.35g of 40%(w/w)benzyltrimethylammonium hydroxide aqueous solution, and dropwise added 17.9g of acrylonitrile at 8 to 15°C, which was then stirred at 10 to 20°C for 1.5 hours. To this reaction mixture were added 1.25mL of hydrochloric acid, 100mL of propanol and 5.05g of water. Thereto was dropwise added 55.0g of sulfuric acid, which was then refluxed for 6 hours. After cooling, to the reaction mixture was added 100mL of water. The organic layer was separated. Thereto was added 50mL of methanol and dropwise added water(50mL) solution of 31.5g of potassium hydroxide, which was then stirred at room temperature for 1.5 hours. To this reaction mixture were added 75mL of toluene and 75mL of water. The aqueous layer was separated, thereto was added 100mL of toluene, the pH was adjusted to 0.9 with 6mol/L hydrochloric acid, and the organic layer was separated. After the solvent was distilled off under reduced pressure, thereto were added 50mL of toluene and 125mL of cyclohexane. The precipitate was collected by filtration to provide 59.6g of 3-(2-(1-benzothiophen-5-yl)ethoxy)propionic acid as white solid form.

The Chemical shift values of ¹H-NMR spectral in CDCl₃ agreed with the values of example 4-1(2).

### Example 4-5

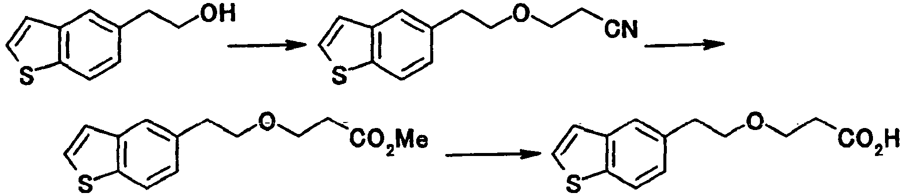

To toluene(260mL) suspension of 260g of 2-(1-benzothiophen-5-yl)ethanol were added 43.8g of 2-propanol and 1.64g of potassium tert-butoxide, which was then stirred for 0.5 hours. After cooling the reaction mixture to 15°C, thereto was dropwise added 116g of acrylonitrile, which was then stirred at 15 to 25°C for 1 hour. To this reaction mixture were added 6.5mL of hydrochloric acid, 520mL of methanol and 78.9g of water. Thereto was introduced 310g of hydrogen chloride at 10 to 25°C, which was then refluxed for 3 hours. After cooling, to the reaction mixture were added 780mL of water and 520mL of toluene, and the organic layer was separated. To the organic layer were dropwise added 260mL of methanol and water(260mL) solution of 164g of potassium hydroxide, which was then stirred at 30 to 35°C for 2 hours. To this reaction mixture was added 260mL of water, and the aqueous layer was separated. To the aqueous layer were added 520mL of toluene and 260mL of water and dropwise added 234mL of hydrochloric acid, and the organic layer was separated. After 390mL of solvent was distilled off under reduced pressure from the organic layer, thereto was added 1040mL of cyclohexane. The precipitate was collected by filtration to provide 326g of 3-(2-(1-benzothiophen-5-yl)ethoxy)propionic acid as white solid form.

The Chemical shift values of ¹H-NMR spectral in CDCl₃ agreed with the values of example 4-1(2).

### Example 4-6

To toluene(360mL) suspension of 180g of 2-(1-benzothiophen-5-yl)ethanol was added 4.22g of aqueous solution of 40%(w/w)benzyltrimethylammonium hydroxide, and dropwise added 8.04g of acrylonitrile at 30°C. After cooling the reaction mixture to 20°C, thereto was dropwise added 53.6g of acrylonitrile, which was then stirred at 15 to 25°C for 2 hours. To this reaction mixture were added 27mL of hydrochloric acid and 180mL of methanol. Thereto was introduced 97g of hydrogen chloride at 10 to 25°C, which was then stirred at 30 to 40°C for 30 minutes and refluxed for 3 hours. After cooling, to the reaction mixture was added 360mL of water, and the organic layer was separated. To the organic layer were dropwise added 180mL of methanol, and water(180mL) solution of 113g of potassium hydroxide, which was then stirred at 30 to 35°C for 2 hours. To the reaction mixture was added 360mL of water, and the aqueous layer was separated. To this aqueous layer was added 360mL of toluene and dropwise added 151mL of hydrochloric acid, and the organic layer was separated. After 126mL of solvent was distilled off under normal pressure from the organic layer, thereto was added 1080mL of cyclohexane. The precipitate was collected by filtration to provide 222g of 3-(2-(1-benzothiophen-5-yl)ethoxy)propionic acid as white solid form.

The Chemical shift values of ¹H-NMR spectral in CDCl₃ agreed with the values of example 4-1(2).

### Example 5-1

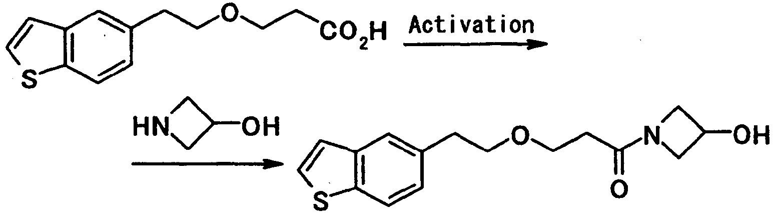

In 15mL of 1,2-dimethoxyethane was dissolved 10.0g of 3-(2-(1-benzothiophen-5-yl)ethoxy)propionic acid. Thereto were added 0.1mL of N,N-dimethylformamide and 5.23g of thionyl chloride, which was then stirred at room temperature for 1.5 hours. This reaction solution was dropwise added to a mixed solution of 50mL of water, 7.19g of sodium hydroxide and 7.69g of 3-azetidinol 1/2 tartrate at 5 to 15°C, which was then stirred at same temperature for 2 hours. Thereto was added 90mL of water. The precipitate was collected by filtration to provide 11.0g of 1-(3-(2-(1-benzothiophen-5-yl)ethoxy)propionyl)azetidin-3-ol as white solid form.
¹H-NMR(CDCl₃) δ value:
2.25-2.35(2H,m),2.96(2H,t,J=7.0Hz),3.65-3.80 (5H,m),3.85-3.95(1H,m),4.05-4.15(1H,m),4.15-4.25(1H,m),4.40-4.50(1H,m),7.19(1H,dd,J=8.3,1.5Hz), 7.27(1H,d,J=5.4Hz),7.40(1H,d,J=5.4Hz),7.62-7.66(1H,m), 7.78(1H,d,J=8.3Hz)

### Example 5-2

To 116mL of toluene was suspended 29.0g of 3-(2-(1-benzothiophen-5-yl)ethoxy)propionic acid. Thereto were added 0.6mL of N,N-dimethylformamide and 14.5g of thionyl chloride, which was then stirred at room temperature for 2 hours. After that, 62mL of solvent was distilled off under reduced pressure. This reaction solution was dropwise added to a mixed solution of 87mL of water, 13.9g of sodium hydroxide and 25.7g of 3-azetidinol 1/2 tartrate at 10 to 20°C, which was then stirred at 20 to 25°C for 1 hour and left unattended overnight. After cooling the reaction solution, the pH was adjusted to 6 with 7mL of acetic acid. After stirring at 10 to 15°C for 1 hour, the precipitate was collected by filtration to provide 31.9g of 1-(3-(2-(1-benzothiophen-5-yl)ethoxy)propionyl)azetidin-3-ol as white solid form.

The Chemical shift values of ¹H-NMR spectral in CDCl₃ agreed with the values of example 5-1.

### Example 5-3

In 75mL of 1,2-dimethoxyethane was dissolved 50.0g of 3-(2-(1-benzothiophen-5-yl)ethoxy)propionic acid. Thereto was added 26.1g of thionyl chloride, which was then refluxed for 2 hours. After cooling, this reaction solution was dropwise added to a mixed solution of 125mL of water, 20.0g of sodium hydroxide and 25.2g of 3-azetidinol hydrochloride at -5 to 10°C, which was then stirred at 0 to 15°C for 30 minutes. Thereto was added 75mL of water, which was heated to 40°C and dissolved. After cooling, the precipitate was collected by filtration to provide 56.5g of 1-(3-(2-(1-benzothiophen-5-yl)ethoxy)propionyl)azetidin-3-ol as white solid form.

The Chemical shift values of ¹H-NMR spectral in CDCl₃ agreed with the values of example 5-1.

### Example 6-1

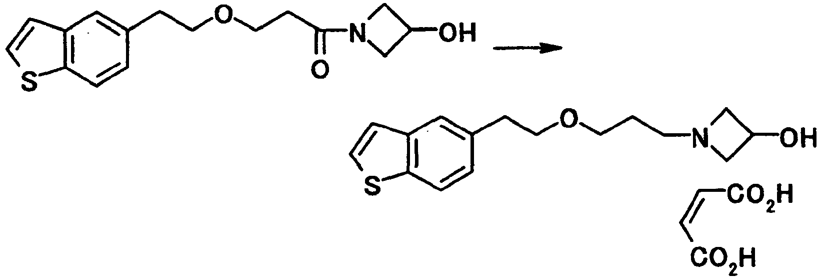

To bis(2-methoxyethyl) ether(5mL) suspension of 1.00g of 1-(3-(2-(1-benzothiophen-5-yl)ethoxy)propionyl)azetidin-3-ol was added 0.37g of sodium borohydride, which was then cooled to 10°C. Thereto was dropwise added 2.49mL of chlorotrimethylsilane at 5 to 10°C for 20 minutes, which was then stirred at room temperature for 2.5h and at 40°C for 4 hours. After cooling, thereto was dropwise added 3.27mL of 6.0mol/L hydrochloric acid, which was then stirred at 70 to 75°C for 30 minutes. To the reaction mixture were added water and ethyl acetate, and the pH was adjusted to 10.0 with 2.0mol/L sodium hydroxide aqueous solution. The organic layer was separated, washed sequentially with water and saturated sodium chloride aqueous solution. Thereto were added anhydrous magnesium sulfate and activated carbon. After insoluble matter was filtered off, the solvent was distilled off under reduced pressure. To the resultant residue was added 0.36g of maleic acid, which was solidified from a mixed solvent(5mL) of ethyl acetate:2-propanol(4:1) to provide 0.72g of 1-(3-(2-(1-benzothiophen-5-yl)ethoxy)propyl)azetidin-3-ol maleate as colorless crystal form.
¹H-NMR(DMSO-d₆) δ value:
1.65-1.75(2H,m),2.93(2H,t,J=6.9Hz),3.14(2H,t,J=7.4Hz), 3.44(2H,t,J=6.0Hz),3.63(2H,t,J=6.9Hz),3.75-3.85(2H,m), 4.15-4.25(2H,m),4.40-4.50(1H,m),6.06(2H,s),7.26 (1H,dd,J=8.3,1.5Hz),7.41(1H,d,J=5.4Hz),7.73(1H,d,J=5.4H z),7.70-7.75(1H,m),7.91(1H,d,J=8.3Hz)

### Example 6-2

To 1,2-dimethoxyethane(5mL) suspension of 1.00g of 1-(3-(2-(1-benzothiophen-5-yl)ethoxy)propionyl)azetidin-3-ol was added 0.37g of sodium borohydride, which was then cooled to 10°C. Thereto was dropwise added 2.49mL of chlorotrimethylsilane at 5 to 10°C, which was then stirred at room temperature for 2.5h and at 40°C for 4 hours. After cooling, thereto was dropwise added 3.27mL of 6.0mol/L hydrochloric acid, which was then stirred at 70 to 75°C for 30 minutes. To the reaction mixture were added water and ethyl acetate, and the pH was adjusted to 10.0 with 2.0mol/L sodium hydroxide aqueous solution. The organic layer was separated, washed sequentially with water and saturated sodium chloride aqueous solution. Thereto were added anhydrous magnesium sulfate and activated carbon. After insoluble matter was filtered off, the solvent was distilled off under reduced pressure. To the resultant residue was added 0.36g of maleic acid, which was solidified from a mixed solvent(5mL) of ethyl acetate:2-propanol(4:1) to provide 0.71g of 1-(3-(2-(1-benzothiophen-5-yl)ethoxy)propyl)azetidin-3-ol maleate as colorless crystal form.

The Chemical shift values of ¹H-NMR spectral in DMSO-d₆ agreed with the values of example 6-1.

### Example 6-3

To tetrahydrofuran(5mL) suspension of 1.00g of 1-(3-(2-(1-benzothiophen-5-yl)ethoxy)propionyl)azetidin-3-ol was added 0.37g of sodium borohydride, and dropwise added tetrahydrofuran(1mL) solution of 0.75mL of trifluoroacetic acid for 30 minutes, which was then refluxed for 2 hours. After cooling, thereto was dropwise added 3.27mL of 6.0mol/L hydrochloric acid, which was then refluxed for 1.5 hours. To the reaction mixture were added water and ethyl acetate, and the aqueous layer was separated. To the aqueous layer was added ethyl acetate, and the pH was adjusted to 10.0 with 20%(w/w) sodium hydroxide aqueous solution. The organic layer was separated, washed sequentially with water and saturated sodium chloride aqueous solution, and dried over anhydrous magnesium sulfate. After insoluble matter was filtered off, the solvent was distilled off under reduced pressure. To the resultant residue was added 0.36g of maleic acid, which was solidified from a mixed solvent(5mL) of ethyl acetate:2-propanol(4:1) to provide 0.62g of 1-(3-(2-(1-benzothiophen-5-yl)ethoxy)propyl)azetidin-3-ol maleate as colorless crystal form.

The Chemical shift values of ¹H-NMR spectral in DMSO-d₆ agreed with the values of example 6-1.

### Example 6-4

To tetrahydrofuran(3mL) suspension of 0.50g of 1-(3-(2-(1-benzothiophen-5-yl)ethoxy)propionyl) azetidin-3-ol was added 0.19g of sodium borohydride, which was then heated to 50°C. Thereto was dropwise added tetrahydrofuran(1mL) solution of 0.46mL of dimethyl sulfate at 50 to 55°C for 10 minutes, which was then stirred at same temperature for 2.5 hours. After cooling, thereto was dropwise added 1.64mL of 6.0mol/L hydrochloric acid, which was then refluxed for 1.5 hours. To the reaction mixture was added ethyl acetate, and the pH was adjusted to 10.0 with 20%(w/w)sodium hydroxide aqueous solution. The organic layer was separated, washed sequentially with water and saturated sodium chloride aqueous solution, and dried over anhydrous magnesium sulfate. After insoluble matter was filtered off, the solvent was distilled off under reduced pressure. To the resultant residue was added 0.18g of maleic acid, which was solidified from a mixed solvent(3.75mL) of ethyl acetate:2-propanol(4:1) to provide 0.49g of 1-(3-(2-(1-benzothiophen-5-yl)ethoxy)propyl)azetidin-3-ol maleate as colorless crystal form.

The Chemical shift values of ¹H-NMR spectral in DMSO-d₆ agreed with the values of example 6-1.

### Example 6-5

To bis(2-methoxyethyl) ether (5mL) suspension of 1.00g of 1-(3-(2-(1-benzothiophen-5-yl)ethoxy)propionyl)azetidin-3-ol was added 0.37g of sodium borohydride, which was then cooled to 10°C. Thereto was dropwise added 2.46mL of 4.0mol/L hydrogen chloride/dioxane at 5 to 15°C for 22 minutes, which was then stirred at same temperature for 30 minutes, at room temperature for 3 hours and at 35 to 40°C for 6 hours. After cooling, thereto was dropwise added 3.27mL of 6.0mol/L hydrochloric acid, which was then stirred at 65 to 70°C for 1.5 hours. To the reaction mixture were added water and ethyl acetate, and the pH was adjusted to 10.0 with 2.0mol/L sodium hydroxide aqueous solution. The organic layer was separated, washed sequentially with water and saturated sodium chloride aqueous solution. Thereto were added anhydrous magnesium sulfate and activated carbon. After insoluble matter was filtered off, the solvent was distilled off under reduced pressure. To the resultant residue was added 0.36g of maleic acid, which was solidified from a mixed solvent(5mL) of ethyl acetate:2-propanol(4:1) to provide 0.86g of 1-(3-(2-(1-benzothiophen-5-yl)ethoxy)propyl)azetidin-3-ol maleate as colorless crystal form.

¹H-NMR in DMSO-d₆ agreed with the values of example 6-1.

### Example 6-6

To 1,2-dimethoxyethane(5mL) suspension of 1.00g of 1-(3-(2-(1-benzothiophen-5-yl)ethoxy)propionyl)azetidin-3-ol was added 0.37g of sodium borohydride, which was then cooled to 10°C. Thereto was dropwise added 2.46mL of 4.0mol/L hydrogen chloride/dioxane at 5 to 15°C for 10 minutes, which was then stirred at same temperature for 1 hour, at room temperature for 3.5 hours and at 35 to 40°C for 6 hours. After cooling, thereto was dropwise added 3.27mL of 6.0mol/L hydrochloric acid, which was then stirred at 65 to 70°C for 1.5 hours. To the reaction mixture were added water and ethyl acetate, and the pH was adjusted to 10.0 with 2.0mol/L sodium hydroxide aqueous solution. The organic layer was separated, washed sequentially with water and saturated sodium chloride aqueous solution. Thereto were added anhydrous magnesium sulfate and activated carbon. After insoluble matter was filtered off, the solvent was distilled off under reduced pressure. To the resultant residue was added 0.36g of maleic acid, which was solidified from a mixed solvent(5mL) of ethyl acetate:2-propanol(4:1) to provide 0.93g of 1-(3-(2-(1-benzothiophen-5-yl)ethoxy)propyl)azetidin-3-ol maleate as colorless crystal form.
¹H-NMR in DMSO-d₆ agreed with the values of example 6-1.

### Example 6-7

To 1,2-dimethoxyethane(70mL) suspension of 20.0g of 1-(3-(2-(1-benzothiophen-5-yl)ethoxy)propionyl)azetidin-3-ol was added 5.46g of sodium borohydride, which was then cooled to 15°C. Thereto was dropwise added 20.6mL of 7.0mol/L hydrogen chloride/1,2-dimethoxyethane at 15 to 20°C for 40 minutes, which was then stirred at room temperature for 1.5 hours and at 53 to 57°C for 4 hours. After cooling, thereto was dropwise added 65.5mL of 6.0mol/L hydrochloric acid, which was then stirred at 65 to 70°C for 1 hour. The reaction mixture was concentrated under reduced pressure, thereto were added 100mL of water and 100mL of ethyl acetate, and the pH was adjusted to 10.0 with 5.0mol/L sodium hydroxide aqueous solution. After the organic layer was separated, washed with 50mL of water, and the pH was adjusted to 1.0 with 6.0mol/L hydrochloric acid. The aqueous layer was separated, to which was added 50mL of ethyl acetate. The pH was adjusted to 10.0 with 5.0mol/L sodium hydroxide aqueous solution. The organic layer was separated, to which was added anhydrous magnesium sulfate. After insoluble matter was filtered off, the solvent was distilled off under reduced pressure. To the resultant residue was added 7.22g of maleic acid, which was solidified from a mixed solvent(100mL) of ethyl acetate:2-propanol(4:1) to provide 19.2g of 1-(3-(2-(1-benzothiophen-5-yl)ethoxy)propyl)azetidin-3-ol maleate as colorless crystal form.

### Example 6-8

To tetrahydrofuran(35.0mL) suspension of 5.00g of 1-(3-(2-(1-benzothiophen-5-yl)ethoxy)propionyl)azetidin-3-ol was added 1.61g of sodium borohydride, and dropwise added tetrahydrofuran(15mL) solution of 2.09g of sulfuric acid at room temperature for 30 minutes, which was then stirred at 48 to 52°C for 7.5 hours. After cooling, thereto was dropwise added 16.4mL of 6.0mol/L hydrochloric acid, which was then refluxed for 1 hour. The reaction mixture was concentrated under reduced pressure, thereto were added water and ethyl acetate, and the pH was adjusted to 9.5 with 5.0mol/L sodium hydroxide aqueous solution. The organic layer was separated, and washed with saturated sodium chloride aqueous solution. Thereto were added anhydrous magnesium sulfate and activated carbon. After insoluble matter was filtered off, the solvent was distilled off under reduced pressure. To the resultant residue was added 1.81g of maleic acid, which was solidified from a mixed solvent(25mL) of ethyl acetate:2-propanol(4:1) to provide 4.82g of 1-(3-(2-(1-benzothiophen-5-yl)ethoxy)propyl)azetidin-3-ol maleate as colorless crystal form.

¹H-NMR in DMSO-d₆ agreed with the values of example 6-1.

### Example 6-9

To tetrahydrofuran(2.38L) suspension of 340g of 1-(3-(2-(1-benzothiophen-5-yl)ethoxy)propionyl)azetidin-3-ol was added 110g of sodium borohydride, and dropwise added tetrahydrofuran(1.02L) solution of 142g of sulfuric acid at room temperature for 1 hour, which was then stirred at 45 to 55°C for 5 hours. After cooling, thereto was added 170mL of acetone and dropwise added 204mL of 36% hydrochloric acid, which was then stirred at room temperature for 3 hours and left unattended overnight. To the reaction mixture was added 1.02L of water, and 3.34L of the solvent was distilled off under reduced pressure. After cooling, thereto was added 0.68L of ethyl acetate, and dropwise added water(0.68L) solution of 147g of sodium hydroxide at 14 to 22°C, which was then stirred at 7 to 15°C for 30 minutes. Insoluble matter was filtered off, washed with 0.34L of ethyl acetate. The filtrate and washings were combined, and the organic layer was separated, washed with 0.68L of water. After to the organic layer was added 2.04L of 2-propanol, 3.01L of the solvent was distilled off under reduced pressure. Thereto were added 1.02L of ethyl acetate and 34g of activated carbon, which was stirred for 20 minutes. Insoluble matter was filtered off and washed with 0.34L of ethyl acetate. The filtrate and washings were combined, thereto was added 116g of maleic acid. After this reaction mixture was heated and dissolved, it was slowly cooled. The precipitate was collected by filtration to provide 376g of 1-(3-(2-(1-benzothiophen-5-yl)ethoxy)propyl)azetidin-3-ol maleate as colorless crystal form.

¹H-NMR in DMSO-d₆ agreed with the values of example 6-1.

### Example 6-10

To tetrahydrofuran(250mL) suspension of 50.0g of 1-(3-(2-(1-benzothiophen-5-yl)ethoxy)propionyl)azetidin-3-ol was added 13.6g of sodium borohydride, and dropwise added 18.5g of sulfuric acid at room temperature for 3 hours, which was then stirred at 45 to 55°C for 4.5 hours. After cooling, thereto was added 15mL of acetone and dropwise added 120mL of 6.0mol/L hydrochloric acid, which was then refluxed for 1 hour. To the reaction mixture was added 150mL of water, and the solvent was distilled off under reduced pressure. Thereto was added 200mL of ethyl acetate, and dropwise added water(100mL) solution of 43.9g of sodium hydroxide at 10 to 21°C. The organic layer was separated, washed with 20% sodium chloride aqueous solution. Thereto were added 50.0g of zeolite and 5.0g of activated carbon, which was then stirred for 20 minutes. Insoluble matter was filtered off and washed with 100mL of ethyl acetate. The filtrate and washings were combined, thereto were added 63mL of ethyl acetate, 75mL of 2-propanol and 17.1g of maleic acid. After this reaction mixture was heated and dissolved, it was slowly cooled. The precipitate was collected by filtration to provide 56.7g of 1-(3-(2-(1-benzothiophen-5-yl)ethoxy)propyl)azetidin-3-ol maleate as colorless crystal form.
¹H-NMR in DMSO-d₆ agreed with the values of example 6-1.

### INDUSTRIAL APPLICABILITY

The process for production of 1-(3-(2-(1-benzothiophen-5-yl)ethoxy)propyl)azetidin-3-ol and salts thereof of the present invention has the following characteristics, (1) safety to human body, (2) low environmental loads and (3) a possibility of mass production, and so forth, therefore, the process of 1-(3-(2-(1-benzothiophen-5-yl)ethoxy)propyl)azetidin-3-ol and salts thereof is useful as industrial manufacturing process.

## Claims

1. A process for production of 1-(3-(2-(1-benzothiophen-5-yl)ethoxy)propyl)azetidin-3-ol or salts thereof **characterized by** subjecting 1-(3-(2-(1-benzothiophen-5-yl)ethoxy)propionyl)azetidin-3-ol to reduction reaction with an addition of activator in the presence of alkali metal borohydride, wherein the activator is a protonic acid.

2. The process for production of 1-(3-(2-(1-benzothiophen-5-yl)ethoxy)propyl)azetidin-3-ol or salts thereof according to claim 1, wherein the activator used is sulfuric acid.

3. The process for production of 1-(3-(2-(1-benzothiophen-5-yl)ethoxy)propyl)azetidin-3-ol or salts thereof according to claim 1 or 2, wherein the alkali metal borohydride used is sodium borohydride.

4. The process for production of 1-(3-(2-(1-benzothiophen-5-yl)ethoxy)propyl)azetidin-3-ol or salts thereof according to claim 2, wherein the amount of sulfuric acid used is 0.5 to 0.6 times mole per mole of the alkali metal borohydride and the process is **characterized by** adding sulfuric acid at 0 to 30°C for 10 minutes to 6 hours, and subsequently reacting at 30 to 70°C.

## Patentansprüche

1. Verfahren zur Herstellung von 1-(3-(2-(1-Benzothiophen-5-yl)ethoxy)propyl)azetidin-3-ol oder Salzen davon, **gekennzeichnet durch** eine Reduktion von 1-(3-(2-(1-Benzothiophen-5-yl)ethoxy)propionyl)azetidin-3-ol unter Zusatz eines Aktivators in Gegenwart von Alkalimetallborhydrid, wobei der Aktivator eine Protonensäure ist.

2. Das Verfahren zur Herstellung von 1-(3-(2-(1-Benzothiophen-5-yl)ethoxy)propyl)azetidin-3-ol oder Salzen davon gemäß Anspruch 1, wobei der Aktivator Schwefelsäure ist.

3. Das Verfahren zur Herstellung von 1-(3-(2-(1-Benzothiophen-5-yl)ethoxy)propyl)azetidin-3-ol oder Salzen davon gemäß Anspruch 1 oder 2, wobei das Alkalimetallborhydrid Natriumborhydrid ist.

4. Das Verfahren zur Herstellung von 1-(3-(2-(1-Benzothiophen-5-yl)ethoxy)propyl)azetidin-3-ol oder Salzen davon gemäß Anspruch 2, wobei die Menge der verwendeten Schwefelsäure die 0,5 bis 0,6-fache Molmenge pro Mol des Alkalimetallborhydrids ist, und das Verfahren **gekennzeichnet ist durch** Zugabe der Schwefelsäure bei 0 bis 30°C über 10 Minuten bis 6 Stunden, gefolgt von Umsetzen bei 30 bis 70°C.

## Revendications

1. Procédé pour la production de 1-(3-(2-(1-benzothiophén-5-yl)éthoxy)propyl)azétidin-3-ol ou de sels de celui-ci, **caractérisé par** le fait de soumettre du 1-(3-(2-(1-benzothiophén-5-yl)éthoxy)-propionyl)azétidin-3-ol à une réaction de réduction avec addition d'un activateur en présence d'un borohydrure de métal alcalin, procédé dans lequel l'activateur est un acide protonique.

2. Procédé pour la production de 1-(3-(2-(1-benzothiophén-5-yl)éthoxy)propyl)azétidin-3-ol ou de sels de celui-ci selon la revendication 1, dans lequel l'activateur utilisé est l'acide sulfurique.

3. Procédé pour la production de 1-(3-(2-(1-benzothiophén-5-yl)éthoxy)propyl)azétidin-3-ol ou de sels de celui-ci selon la revendication 1 ou 2, dans lequel le borohydrure de métal alcalin utilisé est le borohydrure de sodium..

4. Procédé pour la production de 1-(3-(2-(1-benzothiophén-5-yl)éthoxy)propyl)azétidin-3-ol ou de sels de celui-ci selon la revendication 2, dans lequel la quantité d'acide sulfurique utilisée est de 0,5 à 0,6 mole par mole du borohydrure de métal alcalin, et le procédé est **caractérisé par** l'addition d'acide sulfurique à une température de 0 à 30°C pendant 10 minutes à 6 heures, et ensuite par réaction à une température de 30 à 70°C.
